# EUROPEAN PATENT APPLICATION

(11) **EP 3 075 407 A1**
(43) Date of publication of application: **05.10.2016**
(21) Application number: 15162501.9
(22) Date of filing: 02.04.2015
(51) Int. Cl.: A61M 16/10, A61M 16/06, A61M 16/00

(54) **CLINICAL OXYGEN SUPPLY DEVICE, OXYGEN SUPPLY SYSTEM AND METHOD OF OXYGEN SUPPLY**

(71) Applicant: Linde Aktiengesellschaft, 80331 München (DE)
(72) Inventor: Häussermann, Sabine, 82061 Neuried (DE); Forsberg, Peter, 85521 Ottobrunn (DE)
(74) Representative: m patent group

(57) **Abstract**

A automatically operable clinical oxygen supply device (100) adapted to supply supplemental oxygen to a patient, comprising an oxygen inlet section (110) with a first coupling unit (111) adapted to be releasably coupled to a standardized oxygen wall outlet (1), an oxygen outlet section (120) with a second coupling unit (121) adapted to be releasably coupled to a oxygen dispensing device (2) attached to a patient (3), an oxygen flow control section (130) with a communication unit (111) adapted to receive data from an oxygen saturation sensing device (4) attached to the patient (3), and with a regulation unit (112) adapted to regulate an oxygen flow between the oxygen inlet section (110) and the oxygen outlet section (120) on the basis of data received by the communication unit (111) is provided. The automatically operable clinical oxygen supply device (100) further comprises an energy conversion section (140) adapted to generate electrical energy from pressure energy of oxygen supplied to the oxygen inlet section (110), and an energy supply section (150) adapted to provide the electrical energy generated by the energy conversion section (140) to the oxygen flow control section (130). A clinical oxygen supply system (1) and a method of clinical oxygen supply are also part of the present invention.

## Description

The invention relates to a clinical oxygen supply device, to a clinical oxygen supply system, and to a method of clinical oxygen supply according to the precharacterising clauses of the independent claims.

### Prior art

Oxygen supply arrangements for clinical purposes may comprise an oxygen wall outlet to which a manually operable oxygen flow setting device is coupled. The manually operable oxygen flow setting device is classically equipped with a so called rotameter, i.e. with a device measuring the flow rate of oxygen gas in a closed tube as originally described in DE 215 225 A, or with another flow rate indicator.

While, for extensive surgical treatment, a patient in need of oxygen supply is typically intubated and exclusively supplied with oxygen by means of a sophisticated respiratory unit, also termed "ventilator", this is typically not the case in emergency rooms, during recovery of acute respiratory patients and during some forms of critical care treatment where patients are not completely ventilated but still need supplemental oxygen. In the latter cases, patients are typically supplied with the supplemental oxygen directly from the wall outlet under control of the manually operable oxygen flow setting device described above. If at all, only comparatively simple respiratory units are used in such cases, e.g. devices for providing so called bilevel positive airway pressure (BiPAP) or continuous positive airway pressure (CPAP). However, under supplemental oxygen supply without sophisticated ventilators also (smaller) surgical interventions may be performed, especially under local anaesthetics.

If supplemental oxygen is supplied to a patient, the oxygen saturation of the blood of the patient should be measured. Especially in cases of supplemental oxygen supply over longer periods of time, oxygen is conventionally measured intermittently. Oxygen measurement is typically performed non-invasively via a so called oximeter. In oximeters, typically a sensor is placed on a thin and therefore optically translucent part of the patient's body, usually a fingertip or an earlobe, and light of two wavelengths is passed through this body part to a photodetector. The absorbance of each of the two wavelengths is measured, allowing a determination of the absorbance of the pulsing arterial blood alone without interference of peripheral blood.

On the basis of an indication of an oxygen saturation sensing device, a nurse or other personnel responsible for the oxygen supply, in the classical arrangements mentioned, manually regulates the oxygen flow to the patient by turning a valve of the manually operable oxygen flow setting device.

From US 6,142,149 A, a mobile system providing an automatically regulated oxygen supply to a patient is known, comprising an automatically operable oxygen flow setting device carried by the patient. US 5,365,922 A, US 6,470,885 A and US 6,532,958 A also disclose automatically operable oxygen flow setting devices. While, thus, also automatically operable oxygen flow setting devices are known from the prior art, they have never found acceptance in the clinical contexts described above, presumably due to the high effort for establishing completely new oxygen supply arrangements and due to the necessity of changing long-term habits of clinical personnel.

From the unpublished European Patent Application No. 14 180 666.1, a method of upgrading a clinical oxygen supply arrangement adapted to supply supplemental oxygen to a patient, comprising an oxygen wall outlet, a manually operable oxygen flow setting device coupled to the oxygen wall outlet, and an oxygen supply line coupled to the manually operable oxygen flow setting device and adapted to provide oxygen to a patient in need of oxygen supply is known. In this application, it is suggested to provide an oxygen saturation sensing device attachable to the patient and adapted to provide data indicative of an oxygen saturation of the blood of the patient on the basis of a non-invasive measurement thereof. Furthermore, it is suggested to substitute the manually operable oxygen flow setting device by an automatically operable oxygen flow setting device comprising control means adapted to control an oxygen flow through the automatically operable oxygen flow setting device on the basis of the data from the oxygen saturation setting device in an automatic operation mode of the automatically operable oxygen flow setting device.

While such an arrangement provides significant advantages over the prior art by allowing to simply substitute a manually operable oxygen flow setting device by an automatically operable oxygen flow setting device without further amending the oxygen supply infrastructure of a hospital or the like, there is still a need for further improvement, especially in view of an improved autarky of the automatically operable oxygen flow setting device used in such an arrangement.

### Summary of the invention

According to the invention, a clinical oxygen supply device, a clinical oxygen supply system, and a method of clinical oxygen supply according to the independent claims is provided. Preferred embodiments of the invention are subject of the dependent claims and of the description that follows.

As mentioned before, the present invention is used in oxygen supply arrangements wherein a patient is supplied with supplemental oxygen directly from an oxygen wall outlet via an oxygen flow setting device, e.g. by use of a laryngeal mask or a nasal cannula, but without the use of sophisticated ventilators that completely substitute autonomous breathing. The invention thus relates to cases wherein an oxygen flow to the patient is directly influencable via regulation of the oxygen flow setting device, while in ventilators the ventilator itself regulates the oxygen flow.

Conventionally, as mentioned, in such scenarios a manually operable oxygen flow setting device is used. Such a manually operable oxygen flow setting device, as mentioned, may be substituted by an automatically operable oxygen flow setting device. However, the automatic operation of such an automatically operable oxygen flow setting device is generally based on electric energy which, according to the prior art, needs to be provides via either a mains, i.e. via an electrical connection to a supply system of electric energy, or via suitable energy storing devices such as batteries.

Especially in the latter case, however, a disruption in oxygen supply may occur if the batteries are discharged. While this problem may be addressed by providing a warning system informing the patient or clinical personnel of an imminent power failure, a corresponding warning could be ignored, especially when issued during times of restricted attendance, e.g. during weekends. Furthermore, if rechargeable batteries are used, they may be suffer from a fatigue effect due to frequent and/or improper recharging cycles resulting from continuous use. In consequence, to provide sufficient security against power losses, battery packs used in an automatically operable oxygen flow setting device tend to be rather bulky or, if high-capacity batteries are used, expensive. Bulky batteries also may, due to their weight, also exert significant mechanical forces upon an oxygen wall outlet and, at least over time, damage the latter. A rather frequent exchange such battery packs, furthermore, which may be inevitable due to the continuous demand for the automatically operable oxygen flow setting device, may generate significant waste problems.

In case an automatically operable oxygen flow setting device is coupled to the mains, it is necessary to use electrical sockets which may not be provided in proximity to the oxygen wall outlet used to supply oxygen to the automatically operable oxygen flow setting device. Therefore, typically rather unhandy electrical connections have to be made, including cables which may pose tripping and/or disconnection hazards.

The present invention overcomes these problems and suggests a conversion of pressure energy latently present in oxygen supplied to the automatically operable oxygen flow setting device into electric energy that may be used to operate the automatically operable oxygen flow setting device or the means for controlling an oxygen flow contained in such a device.

The present invention is especially based on the previous finding that an exchange of only the existing manually operable oxygen flow setting devices as described above to automatically operable oxygen flow setting devices that are specifically adapted to be exchangeable therewith, leaving the rest of the clinical oxygen supply arrangement untouched, will provide broader acceptance of clinical oxygen supply arrangements provided thereby, and, consequently, allow for a better oxygen supply to patients which is less prone to human error. Conventionally, in the entirely manual method as described above, an operator of a manually operable oxygen flow setting device may, e.g. due to stress during the treatment of emergency cases or surgical treatment, omit to upregulate the oxygen flow or, in some cases with even more severe consequences, forget to downregulate a previously upregulated oxygen flow, thus providing excessive oxygen amounts to the patient.

Conventionally, during the treatment of emergency cases or surgical treatment, the paradigm "as much oxygen as possible" was followed. More modern methods of treatment, however, suggest supplying "titrated oxygen", i.e. provide optimum oxygen saturation, especially in patient groups which are extremely sensitive to excessive oxygen supply, e.g. patients with chronical obstructive lung disease (COPD). In such patients, excessive oxygen supply might result in reduced breathing or the complete stop of breathing. In case of myocardial infarction, seizures or stroke, a high peripheral blood perfusion is needed. Excessive oxygen supply, however, results in the reduction of peripheral blood flow and is therefore contraindicated.

Especially in these modern methods of treatment with tight oxygen titration, a manual operation of entirely manually operable oxygen flow setting devices becomes difficult or even impossible in the hectic environment of a hospital, since it would take time. It is highly likely that an operator, in an entirely manually operation, leaves the optimum range of the oxygen supply for specific types of patients. Completely exchanging existing clinical supply arrangements for establishing improved and automatically controllable systems would however include, as mentioned, high invest costs and/or a change of long-established habits in clinical practice.

The present invention further improves the convenience of a shift from manually to automatically operable oxygen flow setting devices by increasing the autarky of the devices involved. An automatically operable oxygen flow setting device provided according to the present invention is significantly less bulky and less prone to power losses (as compared to an automatically operable oxygen flow setting device including batteries) and/or of significantly less hindrance to clinical personnel and/or patients (as compared to an automatically operable oxygen flow setting device connected to the mains). It thus much closer resembles or even exceeds, in terms of ease of operation, the manually operable oxygen flow setting devices including rotameters which are classically used. Therefore, much broader acceptance in clinical contexts is to be expected, leading to an increase in general patient safety.

### Advantages of the invention

In view of the above, the present invention provides an automatically operable clinical oxygen supply device adapted to supply supplemental oxygen to a patient. In other words, the present invention does, as initially mentioned, not relate to scenarios wherein rather advanced oxygen supply units like ventilators are used, but rather to scenarios wherein patients are supplied with oxygen to support and/or improve autonomous breathing.

The automatically operable clinical oxygen supply device according to the present invention comprises an oxygen inlet section with a first coupling unit adapted to be releasably coupled to a standardized oxygen wall outlet. Via such a first coupling unit, which may also be provided as an exchangeable unit and therefore be adaptable to different oxygen wall outlet standards, the oxygen supply device according to the present invention may be coupled easily to a number of different oxygen supply systems and/or their respective wall outlets. It is also possible to provide an automatically operable clinical oxygen supply device with different coupling units that are supplied as exchange and/or spare parts. In a particularly preferred embodiment, the coupling unit is also adapted to be releasably coupled to an oxygen cylinder, e.g. via a suitable adapter. In this way, e.g. a relocation of a patient from one ward to another or from one hospital to another becomes possible without significant oxygen supply breaks. During transport, the oxygen coupling unit is then releasably coupled to the oxygen cylinder for an uninterrupted oxygen supply.

According to the present invention, the automatically operable clinical oxygen supply device further includes an oxygen outlet section with a second coupling unit which is adapted to be releasably coupled to an oxygen dispensing device which is attached or attachable to a patient. Oxygen dispensing devices useable in the context of the present invention include, for example, nasal cannulas, facial (laryngeal) masks and further units as mentioned. As also mentioned, the context of the present invention is rather the supplemental oxygen supply. Thus, the oxygen dispensing device which is attached or attachable to the patient is typically not embodied as an intubation unit. However, also intubation units are intended to be encompassed by the scope of the present invention.

The automatically operable clinical oxygen supply device of the present invention further comprises an oxygen flow control section. This oxygen flow section is provided to restrict an oxygen flow between the oxygen inlet section and the oxygen outlet section in order to provide a controlled oxygen flow to the patient. As explained below, the oxygen flow control section may also be supplied with data regarding a status and/or an oxygen need of the specific patient, e.g. according to a treatment protocol provided from externally and/or stored in storage means of the oxygen flow control section. The oxygen flow control section of the automatically operable clinical oxygen supply device according to the present invention further comprises a communication unit which is adapted to receive data from an oxygen saturation sensing device which is also attached to the patient. The oxygen saturation sensing device is adapted to measure, via a typically non-invasive measurement, an oxygen saturation of the blood of the patient, according to the principles as mentioned above. The oxygen flow control section of the automatically operable clinical oxygen supply device according to the present invention includes a regulating unit adapted to regulate the oxygen flow between the oxygen inlet section and the oxygen outlet section, and therefore an oxygen amount supplied to the oxygen dispensing device and to the patient, on the basis of the data received by the communication unit which are indicative of the oxygen saturation in the blood of the patient.

According to the present invention, to circumvent the problems as indicated above, the automatically operable clinical oxygen supply device comprises an energy conversion section which is adapted to generate electrical energy from pressure energy of oxygen that is supplied to the oxygen inlet section. Furthermore, according to the present invention, an energy supply section is provided which is adapted to provide the electrical energy generated by the energy conversion section to the oxygen flow control section. As mentioned above, the present invention is essentially based on the finding that conversion of latent energy contained in the oxygen that is supplied to the automatically operable clinical oxygen supply device may be used to generate electric energy and to provide a more reliable and/or convenient energy supply to the automatically operable clinical oxygen supply device and/or its components. Regarding specific advantages of the solution according to the present invention, reference is made to the explanations above.

As the oxygen supply device according to the present invention becomes significantly lighter and less bulky, its weight can be carried by an oxygen wall outlet alone without the need of further support structures. Its handling is therefore significantly improved and no floor or wall space is lost for further fixture elements. This represents a particular advantage in clinical contexts where space is often limited due to the presence of a plurality of further treatment devices. If the weight of the oxygen supply device is carried by the oxygen wall outlet and if no further support structures are necessary, also the blocking of neighboring oxygen wall outlets by such support structures is avoided.

According to a particularly advantageous embodiment of the present invention, the energy conversion section comprises a first energy conversion unit which is adapted to convert the pressure energy of the oxygen supplied to the oxygen inlet section to mechanical energy, and a second energy conversion unit which is adapted to convert the mechanical energy into the electrical energy mentioned above. By providing these components, the present invention may make use of standardized products and/or components, as e.g. used in pneumatic technology. The automatically operable clinical oxygen supply device according to the present invention, therefore, allows for a particularly reliable operation by using standardized and well-tested equipment.

It is particularly advantageous if the first energy conversion unit used in the energy conversion section of the automatically operable clinical oxygen supply device according to the present invention is provided as a pneumatic motor and/or an expansion turbine which is adapted to be operated by expansion of the oxygen supply by the oxygen inlet section. Especially pneumatic motors are well-tested and reliable components used in pneumatic technology. A pneumatic motor is a type of motor which provides mechanical energy by expanding a compressed fluid. While pneumatic motors are, in classical pneumatic technology, typically operated by expanding air, they are likewise usable for expanding oxygen and/or oxygen-rich fluids.

In this context, it should be noted that, if the present application uses the term "oxygen", this term also includes oxygen-rich fluids which do not entirely consist of oxygen. The term "oxygen" according to the present application, therefore, also includes fluids which are enriched in oxygen, "enriched" meaning an oxygen content which is significantly above the oxygen content of atmospheric air, i.e. above 25%, 50% and/or 75% by volume of oxygen.

Pneumatic motors are generally adapted to convert pressure energy to mechanical energy by generating either linear or rotary motion. Linear motion may be generated by using e.g. a diaphragm or piston actuator, while rotary motion may be generated by for example a vane type air motor or a piston air motor. To convert linear motion to rotational motion, if necessary, units well-known in the art may be used. Expansion turbines which may also be used as the first energy conversion unit according to the present invention are well-known to the skilled person.

It is further advantageous if the second energy conversion unit used in the energy conversion section of the automatically operable clinical oxygen supply device according to the present invention is provided as an electric generator which is adapted to be rotated or otherwise actuated by the mechanical energy provided by the first energy conversion unit. The generator (also referred to as "alternator") according to the present invention may, for example, be embodied as a linear or a rotational generator. If, for example, a pneumatic motor generating a linear motion as explained above is used as the first energy conversion unit, the second energy conversion unit may be embodied as a linear generator. Linear generators are commonly known to the skilled person and typically resemble linear motors which are operated to generate electric energy from mechanical energy instead of generating mechanical energy from electric energy. If, on the other hand, the first energy conversion unit is provided as a pneumatic motor and/or an expansion turbine generating rotational motion, the second energy conversion unit is preferably provided as a rotational electric generator. In both cases, a suitable mechanic coupling, i.e. suitable shafts and/or rods are provided between the first energy conversion unit and the second energy conversion unit of the energy conversion section of the automatically operable clinical oxygen supply device according to the present invention.

A particularly preferred embodiment of the automatically operable clinical oxygen supply device provided according to the present invention includes that the energy supply section comprises an energy buffering unit which is adapted to buffer the electric energy generated by the energy conversion section. In this way, the electric energy supplied to the oxygen flow control section of automatically operable clinical oxygen supply device according to the present invention may e.g. be smoothed and/or short-time energy losses may be bridged. For example, if an automatically operable clinical oxygen supply device according to the present invention is, for a short time, disconnected from the oxygen wall outlet, e.g. to relocate a patient, the energy buffering unit in the energy supply section assures that e.g. memory contents of the oxygen flow control section are not lost. The automatically operable clinical oxygen supply device according to the present invention does thus not have to be reprogrammed and/or reparameterised if short-term oxygen supply disruptions occur. In this embodiment, it is especially preferred that the energy buffering unit is provided in the form of one or more rechargeable batteries and/or (super-)capacitors. While, in the description above, it is pointed out that an automatically operable clinical oxygen supply device according to the present invention is particularly advantageous because no bulky battery packs have to be provided, such a device may still be of significant advantage even if an energy buffering unit is provided. Such an energy buffering unit generally needs to bridge only short-term oxygen supply interruptions and therefore the batteries and/or capacitors are significantly less bulky and costly. A supercapacitor, which is generally known from the prior art, is especially preferred due to its large capacitance. A supercapacitor bridges the gap between classical electrolytic capacitors and rechargeable batteries. For further details and advantages, reference is made to general literature in the field.

According to particularly preferred embodiment of the present invention, the energy buffering unit provided in the energy supply section of the automatically operable clinical oxygen supply device according to the present invention is adapted to be externally precharged and/or recharged. An externally precharged and/or recharged energy buffering unit may be used as a back up energy supply which may also be provided to ensure operation independently from the electrical energy converted by the energy conversion section. By providing an energy buffering unit correspondingly, a more secure and particularly failsafe operation of the device according to the present invention may be achieved.

It is particularly advantageous if the energy supply section comprises energy status information means which are adapted to inform a user regarding an amount of electrical energy generated by the energy conversion section and/or available to the energy supply section. For example, if an energy buffering unit is provided in the energy supply section, the energy status information means may display a charge state of the buffering unit. For example, if the automatically operable clinical oxygen supply device according to the present invention recently has been decoupled from the oxygen wall outlet, and therefore the energy buffering unit is not yet fully and/or sufficiently charged, the energy status information means may display such information and thus disadvise a user from decoupling the automatically operable clinical oxygen supply device according to the present invention from the oxygen outlet again. For example, the energy states information means may also provide information to a user regarding the amount of electrical energy presently generated, this amount relating directly to pressure to the oxygen supply to the automatically operable clinical oxygen supply device. If, for example, this electrical energy is below a certain threshold, it may be concluded that the oxygen pressure is too low, probably also for a sufficient supply to the patient. Therefore, the energy status information means may also serve as an indication of the oxygen pressure. They may thus function as a second (redundant) security instance, besides a pressure sensor which is separately provided. The energy status information means, due to the functions as explained above, may also serve as the only pressure sensing unit, allowing for a simpler construction of the automatically operable clinical oxygen supply device according to the present invention.

According to a further advantageous embodiment of the present invention, the energy supply section may further comprise an energy supply port which is adapted to supply the electric energy generated by the energy conversion section, or a part thereof, also to the oxygen saturation sensing device. Such sensing devices are typically, but not necessarily, connected to the automatically operable clinical oxygen supply device according to the present invention via sensing cables. It is therefore possible without using further cables to supply electric energy to such oxygen saturation sensing devices. Also an oxygen saturation sensing device may therefore be constructed in a less bulky form as no batteries have to be provided.

The present invention also relates to clinical oxygen supply system which includes a standardized oxygen wall outlet adapted to be supplied with pressurized oxygen, an oxygen dispensing device and an oxygen saturation sensing device attachable to a patient. According to the present invention, such an oxygen supply system includes an automatically operable clinical supply device explained above.

Furthermore, the present invention relates to a method of clinical oxygen supply which includes the use of an automatically operable clinical oxygen supply device as explained above and/or a clinical oxygen supply system as mentioned. Both the clinical oxygen supply system and the method of clinical oxygen supply provided according to the present invention take profit of the advantages mentioned above, to which, therefore, specific reference is made.

Further features and advantages of the automatically operable oxygen flow setting device, of the oxygen supply system, and of the oxygen supply method according to embodiments of the present invention are summarized and discussed below.

As mentioned, the automatically operable oxygen flow setting device according to the present invention comprises a communication unit adapted to communicate at least with the oxygen saturation sensing device. Under a "communication", according to the language used herein, any means of data transfer between two units is to be understood. Communication especially includes mono- and bidirectional data exchange. This means that also simply sending of data indicative of an oxygen saturation of the blood of the patient from the oxygen saturation sensing device attached to the patient to the automatically operable oxygen flow setting device is a form of communication. A communication, according to the present invention, may be realized via different communication protocols, media and/or layers, e.g. via Bluetooth, USB connections, Ethernet, conventional serial links and the like. A "communication" may also include the supply of electric energy.

Most preferably, the communication of the automatically operable oxygen flow setting device according to the present invention comprises the use of at least one receiver which is adapted to wiredly and/or wirelessly receive at least the data from the oxygen saturation sensing device which are indicative of the oxygen saturation of the blood of the patient. At least one receiver may also be adapted to receive data from other units provided in a clinical setup, e.g. handheld devices adapted to input data, especially patient data nor treatment instructions, other oxygen supply arrangements and/or monitoring devices, as also explained below.

Correspondingly, the communication may also include the use of at least one sender which is adapted to wiredly and/or wirelessly send data to at least a monitoring unit and/or at least one other unit provided in the clinical setup. A monitoring unit provided according to a preferred embodiment of the present invention may be adapted to monitor the status of a number of automatically operable oxygen flow setting devices and/or their electrical status.

Such a monitoring unit may especially be used to detect any malfunction and/or unexpected behaviour of an automatically operable oxygen flow setting device and/or unexpected events relating to a status of one or more patients, but also (imminent) power loss. Correspondingly, a preferred embodiment of the present invention includes that the data the at least one sender is adapted to send is indicative of a status of the patient and/or of the automatically operable oxygen flow setting device. By providing a correspondingly adapted sender, it is possible for the clinical personnel to immediately react, e.g. in unexpected and/or emergency situations or power losses.

In this context, it is preferred that the automatically operable oxygen flow setting device is further adapted to also be operated manually in at least one manual operation mode. A manual operation mode may for example be chosen if an unexpected status of the patient and/or of the automatically operable oxygen flow setting device is registered by the monitoring unit described above. The monitoring unit as described above may in this case also be adapted to monitor a signal which continuously sent by the automatically operable oxygen flow setting device. If such a signal, corresponding to e.g. a known dead-man's signal, is absent, the monitoring device may deduce that the automatically operable flow setting device is unresponding and/or malfunctioning. In such cases, the monitoring device may issue a signal indicative of a malfunction of the automatically operable oxygen flow setting device and instruct the personnel to perform a manual operation of the automatically operable oxygen flow setting device.

In other words, it is preferred that the at least one manual operation mode is initiated if a determination is made that the automatically operable oxygen flow setting device is malfunctioning, a power loss is imminent, and/or that the status of the patient is an unexpected one. Consequently, a non responsiveness and/or malfunctioning of the automatically operable oxygen flow setting device may be detected early and an immediate action may be taken, minimizing the risk of harm to the patient.

As explained above, the determination that the automatically operable oxygen flow setting device is malfunctioning is preferentially made on the basis of data sent by the automatically operable flow setting device. Especially, if such data are implausible and/or cease to be sent, a corresponding determination may be made.

According to a preferred embodiment of the present invention, the automatically operable oxygen flow setting device comprises a memory unit adapted to store patient and/or treatment data relating to the patient and/or a history of data provided by the oxygen saturation setting device. A corresponding memory unit may especially comprise a fixed or detachable memory device, e.g. fixed flash memory and/or a removable flash memory card or an USB key which may be inserted in a corresponding memory unit. On a corresponding memory device of the memory unit, the mentioned data may be stored, especially in encrypted form to meet privacy protection prerequisites. In such a memory unit and/or a corresponding memory device, the whole treatment history of the treatment of one or more patients may be stored and provided for later documentation. Possible treatment errors or unexpected events in the treatment may in this way be identified and provided as a basis for further treatment. It is also possible to send corresponding data to an external device, especially via the previously mentioned communication unit, to store such data in the external device for documentation and/or any other further use.

The automatically operable oxygen flow setting device provided according to the present invention may especially include a proportional-integral-derivative (PID) controller, as generally known in the field of oxygen supply.

The automatically operable oxygen flow setting device provided according to the present invention may especially be adapted to store treatment data relating to the patient, e.g. including a specific oxygen demand of the patient to avoid excessive oxygen supply, as mentioned above.

According to a further preferred embodiment of the invention, the automatically operable oxygen flow setting device may be adapted to correlate the patient and/or treatment data mentioned above with the data provided by the oxygen saturation sensing device indicative of the oxygen saturation of the blood of the patient. If e.g. a discrepancy between the latter and the patient and/or treatment data is identified and the discrepancy exceeds a predefined threshold for more than a predetermined time, a signal, as explained above, to a monitoring unit may be issued. In this case, the monitoring unit may initiate a manual operation of the automatically operable oxygen flow setting device, leaving the ultimate decision on how to proceed to human skill.

Further advantages of the present invention are explained with reference to the appended drawings which illustrate an embodiment of the present invention.

### Short description of the drawing

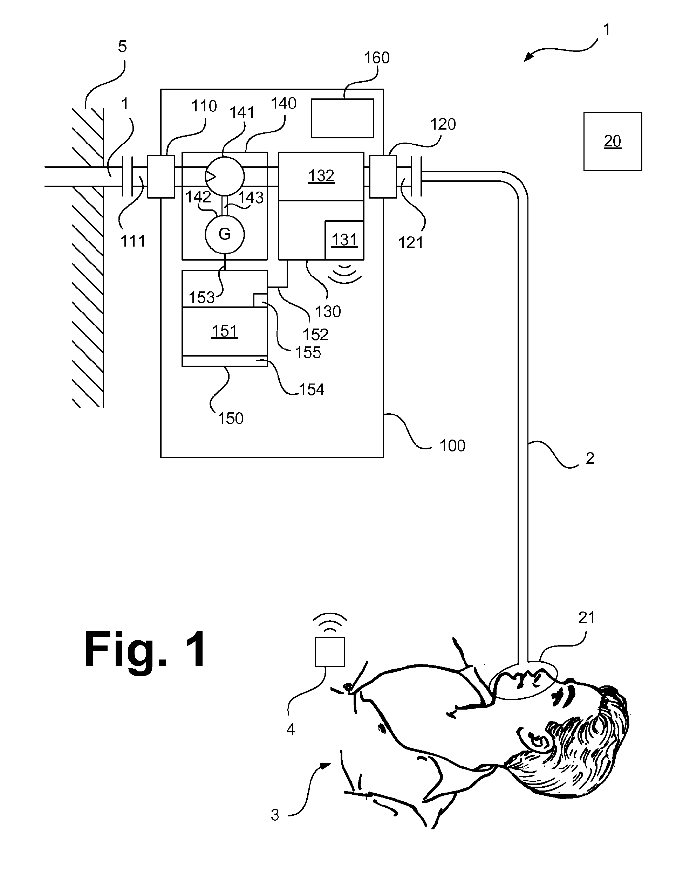

Figure 1 shows an automatically operable clinical oxygen supply device in a clinical oxygen supply system according to a preferred embodiment of the invention.

### Embodiment of the invention

In Figure 1, an automatically operable clinical oxygen supply device is indicated with 100. The automatically operable clinical oxygen supply device 100 may form part of a clinical oxygen supply system 1, as explained above.

The automatically operable clinical oxygen supply device 100 comprises an oxygen inlet section 110 adapted to receive oxygen and an oxygen outlet section 120 adapted to supply oxygen. The oxygen inlet section 110 comprises a first coupling unit 111 which is adapted to be releasably coupled to a standardized oxygen wall outlet 1, e.g. with suitable screw, push-fit, press-fit and/or bayonet coupling means. The oxygen wall outlet 1 is provided in a wall 5, e.g. of clinical ward.

The oxygen outlet section 120, on the other hand, comprises a second coupling unit 121 which is adapted to be releasably coupled to an oxygen dispensing device 2 which can, as shown in Figure 1, be coupled to a patient 3. In the example shown, the oxygen dispensing device 2 comprises a facial mask 21 but other suitable means of oxygen supply to a patient 3 may be provided, e.g. a nasal cannula.

The patient 3 is further equipped with an oxygen saturation sensing device 4 which may operate according to the principles as explained above. In the example shown in Figure 1, the oxygen saturation sensing device is wirelessly coupled to a communication unit 131 of an oxygen flow control section 130 of the automatically operable oxygen supply device 100. However, also a wired connection is possible, such a wired connection also allowing an oxygen saturation sensing device to be supplied with electric energy from the automatically operable clinical oxygen supply device 100. The oxygen flow control section, of which the communication unit 131 is part of, is adapted to regulate an oxygen flow between the oxygen inlet section 110 and the oxygen outlet section 120 via suitable means not shown in detail. The oxygen flow is regulated by the regulation unit 132 on the basis of data received by the communication unit 131, i.e. on the basis of an oxygen saturation of the blood of the patient 3 as measured by the oxygen saturation sensing device 4.

The present invention, in the embodiment shown in Figure 1, includes providing an energy conversion section 140 which is adapted to generate electric energy from pressure energy of oxygen supplied to the oxygen inlet section 110. For this purpose, the energy conversion section in the example shown comprises a first energy conversion unit 141 which may be, as mentioned, be embodied as a pneumatic motor. The first energy conversion unit 141 may be coupled, via a shaft 143, to a second energy conversion unit 142, e.g. an electrical generator. The automatically operable clinical oxygen supply device 100 further includes an energy supply section 150 which is, e.g. via a energy supply line 152, adapted to provide electrical energy generated by the energy conversion section 140 to the oxygen flow control section 130. The energy supply section 150 may, via a further line 153, be connected to the energy conversion section 140. The energy supply section 150 may include an energy buffering unit 151 as explained above which is adapted to buffer the electric energy generated by the energy conversion section 140. The energy supply section 150 may also comprise energy status information means 154, the function of which was already explained in detail above. An energy supply port may also form part of the energy supply section, the energy supply port being adapted to supply the electric energy generated by the energy conversion section 140 also to the oxygen saturation sensing device 5, if the latter is wiredly connected to the automatically operable clinical oxygen supply device according to the present invention.

Furthermore, the automatically operable clinical oxygen supply device may also include a manual operation section 160 which may be adapted to manually operate the oxygen flow control section 130 at least in case of a malfunction of, and/or a lack of electrical energy available to, the energy supply section 150. The manual operation section 160 may also be adapted to be supplied with clinical data, e.g. patient data regarding the status of the patient 3 and/or a treatment protocol. The manual operation section 160 may also be connected to further devices, e.g. via a wired connection or wirelessly. The automatically operable clinical oxygen supply device 100 according to the embodiment shown may therefore also be remotely operable, e.g. from a central terminal and/or a corresponding unit which is indicated with 20.

## Claims

1. An automatically operable clinical oxygen supply device (100) adapted to supply supplemental oxygen to a patient, comprising an oxygen inlet section (110) with a first coupling unit (111) adapted to be releasably coupled to a standardized oxygen wall outlet (1), an oxygen outlet section (120) with a second coupling unit (121) adapted to be releasably coupled to an oxygen dispensing device (2) attached to a patient (3), an oxygen flow control section (130) with a communication unit (131) adapted to receive data from an oxygen saturation sensing device (4) attached to the patient (3), and with a regulation unit (132) adapted to regulate an oxygen flow between the oxygen inlet section (110) and the oxygen outlet section (120) on the basis of the data received by the communication unit (131), **characterized by** an energy conversion section (140) adapted to generate electrical energy from pressure energy of oxygen supplied to the oxygen inlet section (110), and an energy supply section (150) adapted to provide the electrical energy generated by the energy conversion section (140) to the oxygen flow control section (130).

2. A device (100) according to claim 1, wherein the energy conversion section (140) comprises a first energy conversion unit (141) adapted to convert the pressure energy of the oxygen supplied to the oxygen inlet section (110) to mechanical energy, and a second energy conversion unit (142) adapted to convert the mechanical energy into the electrical energy.

3. A device (100) according to claim 2, wherein the first energy conversion unit (141) is provided as a pneumatic motor and/or an expansion turbine adapted to be operated by expansion of the oxygen supplied to the oxygen inlet section (110).

4. A device (100) according to any one of claims 2 or 3, wherein the second energy conversion unit (142) is provided as an electric generator adapted to be operated by the mechanical energy provided by the first energy conversion unit (141).

5. A device (100) according to any one of the preceding claims, wherein the energy supply section (150) comprises an energy buffering unit (151) adapted to buffer the electric energy generated by the energy conversion section (140).

6. A device (100) according to claim 5, wherein the energy buffering unit (151) is provided as one or more rechargeable batteries and/or supercapacitors.

7. A device (100) according to any one of claims 6 or 7, wherein the energy buffering unit (151) is adapted to be externally precharged and/or recharged.

8. A device (100) according to any one of the preceding claims, wherein the energy supply section (150) comprises energy status information means (154) adapted to inform a user regarding an amount of electrical energy generated by the energy conversion section (140) and/or available to the energy supply section (150).

9. A device (100) according to any one of the preceding claims, wherein the energy supply section (150) further comprises an energy supply port (155) adapted to supply the electric energy generated by the energy conversion section (140) also to the oxygen saturation sensing device (4).

10. A device (100) according to any one of the preceding claims, comprising a manual operation section (160) adapted to manually operate the oxygen flow control section (130) at least in case of a malfunction of, and/or a lack of electrical energy available to, the energy supply section (150).

11. A clinical oxygen supply system (1) including a standardized oxygen wall outlet (1) adapted to be supplied with pressurized oxygen, an oxygen dispensing device (2) and an oxygen saturation sensing device (4) attachable to a patient (3), and **characterized by** an automatically operable clinical oxygen supply device (100) according to any one of the preceding claims.

12. A method of clinical oxygen supply, **characterized by** the use of an automatically operable clinical oxygen supply device (100) according to any one of claims 1 to 10 and/or a clinical oxygen supply system (1) according to claim 11.
